# EUROPÄISCHE PATENTSCHRIFT

(11) **EP 1 721 572 B1**
(45) Veröffentlichungstag und Bekanntmachung des Hinweises auf die Patenterteilung: **15.05.2013**
(21) Anmeldenummer: 06090073.5
(22) Anmeldetag: 09.05.2006
(51) Int. Cl.: A61B 5/11, A61H 3/00, A63B 24/00

(54) **Gerät zur Haltungs- und/oder Bewegungskorrektur von Körperteilen**
Device for posture control and/or movement control of body parts
Dispositif de contrôle de la position corporelle et/ou du mouvement de parties de corps

(30) Priorität: 09.05.2005 DE 102005022005
(43) Veröffentlichungstag der Anmeldung: 15.11.2006
(73) Patentinhaber: Gutmann, Anna, 13347 Berlin (DE)
(72) Erfinder: Gutmann, Anna, 13347 Berlin (DE); Gutmann, Dindia, 13347 Berlin (DE); Strobel, Kerstin, 23879 Mölln (DE)
(74) Vertreter: Vonnemann, Gerhard

(56) Entgegenhaltungen:
- EP-A- 0 993 845
- EP-A- 1 123 686
- CA-A1- 2 525 548
- DE-A1- 10 124 242
- US-A- 5 469 861
- US-A- 6 059 576
- US-A- 6 066 075

## Beschreibung

Die Erfindung betrifft ein tragbares Gerät für eine Bewegungs- und/oder Haltungskomektur von gefühlsarmen Körperteilen und von partiell gelähmten Personen zur Verbesserung eines Haltungs- und/oder Gangbildes mit mindestens einem mit dem zu kontrollierenden Körperteil verbundenen Sensor dessen zeitkontinuierliches Signal über eine erste Signalübertragungsstrecke einer Signalaufbereitungs- und -auswerteeinheit aufgeschaltet ist und die Mittel zur Umwandlung eines Signals in ein Reizsignal aufweist, das über eine zweite Signalübertragungsstrecke mit mindestens einem taktilen und/oder elektrostimulierenden Reizgeber verbunden ist, der einen Reiz an dem zu kontrollierenden Körperteil erzeugt und so die Haltung und/oder Bewegung des Körperteiles oder der partiell gelähmten Person korrigiert.

Personen mit gefühlsarmen Körperteilen und partiell gelähmte Personen können im Allgemeinen stehen und/oder gehen. Je nach Art und Ausprägung der Behinderung unterscheidet sich das Gangbild von Person zu Person. Auch wenn Nervenbahnen und bestimmte Nervenzentren defekt sind, so sind die Muskeln dieser Personen jedoch intakt. Sie sind aber nur beschränkt steuerbar. Mit der Zeit bilden sich die Muskeln auf Grund fehlender Beanspruchung zurück, was wiederum zu fortschreitenden Bewegungsstörungen führt.

Grundsätzlich müssten Personen mit einer partiellen Lähmung zumindest nach einem gewissen Training zu ganz normalen Gehbewegungen fähig sein. Die fehlende Muskelkraft in den geschädigten Körperteilen brauchte im Grunde normalen Gehbewegungen nicht im Wege zu stehen: die Knochen des gestreckten Beines funktionieren wie eine Krücke und könnten als solche benutzt werden. Muskelkraft wird dabei kaum benötigt.

Werden Personen mit dieser Behinderung gebeten, sich gerade hinzustellen oder gerade zu gehen, ohne ihnen die Möglichkeit einer Kontrolle von außen zu geben, so werden sie mehr oder weniger krumm stehen oder gehen bzw. humpeln. Werden sie danach gefragt, wie sie die Qualität ihres Ganges selber beurteilen würden, so werden sie im Allgemeinen sagen, dass sie gerade und ausgeglichen gestanden bzw. gegangen sind. Wird ihre Körperhaltung derart korrigiert, dass sie objektiv gerade stehen, so werden sie aussagen, dass sie krumm hingestellt wurden.

Subjektiv fühlt sich also für eine partiell gelähmte Personen oder eine Person mit gefühlsarmen Körperteilen ihr krummer Gang bzw. ihre krumme Haltung als ausgeglichen an, genauso wie sich eine objektiv gerade Haltung für sie subjektiv schief anfühlt.

Diese Beobachtung verdeutlicht, dass neben dem Gleichgewichtsorgan, auch die subjektive Eigenwahrnehmung bezüglich der Symmetrie der Muskelspannungen beider Körperhälften entscheidend ist für die Fähigkeit, eine aufrechte Haltung einzunehmen bzw. für ein aufrechtes, ausgeglichenes Gangbild. Die betreffenden Personen weisen eine Unsymmetrie in der Eigenwahrnehmung bezüglich ihrer Haltung und ihres Ganges auf (Sensibilitätsstörung).

Das Erlernen eines normalen Ganges/einer normalen Körperhaltung ist für halbseitig gelähmte Personen und insbesondere für betroffene Kinder extrem wichtig, weil die Sekundärschäden einer falschen Haltung oder eines falschen Ganges oft schwerer wiegen, als die Lähmung selber: Irreparable Gelenkschädigungen und zerschlissene Wirbelkörper machen bald jedes Gehen unmöglich und binden die Betroffenen nicht selten an den Rollstuhl. Diese Immobilität führt dann zu weiteren Beschwerden - ganz abgesehen von den sozialen Problemen, die diese Schäden nach sich ziehen.

Vorrichtungen zur Überwachung und Kontrolle der Bewegung von Körperteilen sind bereits in unterschiedlicher Ausführung bekannt.

Die in der WO 02095714 A1 beschriebene Erfindung betrifft ein Verfahren zur Haltungs- und/oder Bewegungskontrolle mindestens einer Person mit Hilfe eines Trainingsprogramms. Es eignet sich insbesondere zum Erlernen bestimmter Sportarten, oder eines Musikinstrumentes. Mit Hilfe von Sensoreinrichtungen werden haltungsrelevante Daten erfasst. Diese Daten werden synchronisiert, zusammengefasst und gefiltert. Das Ergebnis dieser Auswertung wird mit den Werten einer Expertendatenbank verglichen. Das Resultat steuert ein Wiedergabegerät, das seinerseits dem Probanden akustische und/oder optische Informationen über den Stand des Trainingsprogramms übermittelt.

Dieses technisch sehr aufwendige Verfahren ist nicht in der Lage, konkrete Informationen über den Entstehungsort und Grad von Haltungs- und/oder Bewegungsfehlern zu übermitteln und diese zu korrigieren.

Die WO 95/11730 offenbart ein Verfahren und eine Einrichtung für eine sensorische Kontrolle für Bewegungsabläufe, wobei eine Information des Probanden auf akustischem Weg erfolgt. Dieses Gerät sieht neben Beschleunigungssensoren auch Geschwindigkeits- und Raumlage-/Raumveränderungssensoren vor und ist damit in der Lage, genauere Informationen über die Stellung einzelner Gliedmaßen zu geben. Die Verwendung eines Speichers für Vorgaben mit denen die Messwerte in Beziehung gesetzt werden, macht den Aufbau des Gerätes ähnlich aufwendig, und damit störanfällig und teuer wie das vorher genannte Verfahren. Von Nachteil ist, dass die Einrichtung eine zeitnahe und interaktive Bewegungskorrektur nicht ermöglicht und dass Bewegungen von gesunden und kranken Körperteilen nicht unabhängig voneinander gemessen und auch nicht unabhängig von einander angesprochen werden.

Aus US-A-6 059 576 geht ein Gerät zur Bewegungs- und Haltungskorrektur von Körperteilen hervor, bei dem mittels eines Sensors ein Signal über eine erste Signalübertragungsstrecke einer Signalaufbereitungs- und -auswerteeinheit aufgeschaltet ist, die Mittel zur Umwandlung des Signals in ein Reizsignal aufweist, dass bei Überschreiten eines vorgegebenen Richtwertes über eine zweite Signalübertragungsstrecke mit einem Reizgeber verbunden ist, der einen Reiz am zu kontrollierenden Körperteil erzeugt.

Diese technische Lösung ist für Personen mit verletzten Körperteilen vorgesehen und hat daher nur einen begrenzten Anwendungsbereich.

Dieses Gerät setzt für die Durchführung der Haltungskorrektur ein bewusstes Handeln der betreffenden Person voraus. Es ist insbesondere geeignet als Überwachungsgerät in der Industrie, aber auch für sportliche und medizinische, insbesondere physiotherapeutische Zwecke, die der Kontrolle bei sich wiederholenden körperlichen Bewegungen bedürfen.

Bei partiell gelähmten Personen und Personen mit gefühlsarmen Körperteilen sind jedoch die Nervenbahnen, die die Bewegung des betreffenden Körperteiles steuern, geschädigt. Eine bewusste ergebnisorientierte Steuerung der gelähmten Gliedmaßen ist diesen Personen objektiv nicht möglich. Daher ist das Gerät gemäß US-A-6 059 576 für partiell gelähmte Personen nicht geeignet. Bei Anwendung dieses Gerätes zur Korrektur derartiger Körperteile sind vielmehr gefährliche gesundheitsschädigende Fehlreaktionen der betreffenden Person und deren Verunsicherung nicht ausgeschlossen.

Aus US-A-6 066 075 geht ein Gerät zur direkten Kontrolle von Körperbewegungen hervor, dass insbesondere für das Training und die Ausbildung von Sportlern und Armeeangehörigen bei Simulierung realer Sport- und Kampfsituationen geeignet ist. Mittels Sensoren werden Körperpositionen ermittelt und in Abhängigkeit von simulierten Außenbedingungen auch Druck-, Wärme- und Geschwindigkeitswerte erfasst und dem Benutzer mitgeteilt.

Messungen an den Beinen der betreffenden Person sind jedoch nur eingeschränkt möglich, da zum Beispiel bei dem Anwinkeln eines Beines nicht unterschieden wird, ob das Anwinkeln des Beines ohne äußere oder mit äußerer Belastung vorgenommen wird.

EP-A-0 993 845 beschreibt ein System zur Überwachung der Haltung des Benutzers an einem Trainingsgerät. Es handelt sich hier um ein nicht tragbares Gerät, da das System unmittelbar mit dem Trainingsgerät verbunden ist. Es dient erfindungsgemäß der Überwachung des Benutzers während des Trainings um insbesondere durch Überbeanspruchung verursachte Schäden an Gelenken, Bändern und Muskeln vorzubeugen. Zu diesem Zweck sind Sensoren am Körper des Benutzers und am jeweiligen Trainingsgerät anzubringen. Gewonnene Messwerte werden mit gespeicherten vorgegebenen Daten verglichen und die Resultate dem Benutzer visuell und/oder akustisch mitgeteilt. Vibratoren können, ohne dass deren Signale modulierbar sind, den Benutzer zu einer Korrektur seiner Haltung animieren.

Die US-A-5 469 861 offenbart ein Gerät, das für Patienten mit Hals- oder Rückenwirbelverletzungen einsetzbar ist. Mit Hilfe dieses Gerätes soll Personen mit Hals- oder Rückenwirbelverletzungen Schutz vor falschen Bewegungen gegeben werden. Eine Einheit des Gerätes kontrolliert zu diesem Zweck die Haltung der Wirbelsäule der betreffenden Person und zeichnet dies auf, indem je ein Sensor am Hals und einer an der Taille befestigt wird. Eine zweite Einheit löst bei Überschreitung vorgegebener Grenzwerte jedoch nur zeitverzögert Alarm aus, der zur Bewegungskorrektur auffordern soll.

Die Erfindung gemäß EP-A-1 123 686 betrifft eine Vorrichtung zur Darstellung einer Bewegungsabweichung gegenüber einer Referenzbewegung durch Vergleich der der ausgeführten Bewegung mit der Referenzbewegung. Sie soll dem Erlernen bestimmter Bewegungsabläufe dienen. Zu diesem Zweck werden Bewegungen optisch digital erfasst und diese mittels eines Prozessors mit einer digitalisierten Referenzbewegung überlagert, um die Bewegungsabweichung sichtbar zu machen. Mittels eines Druckimpulse generierenden Akktuators sollen betreffende Körperteile zu einer gewünschten Bewegung angeregt werden. Die Vorrichtung ist jedoch nicht geeignet, das vegetative Nervensystem zu spontanen Reaktionen anzuregen. Außerdem sind mit der Vorrichtung Messungen an den Beinen nur eingeschränkt möglich, da mit dieser Vorrichtung zum Beispiel bei dem Anwinkeln eines Beines nicht unterschieden wird, ob das Anwinkeln des Beines ohne äußere oder mit äußerer Belastung vorgenommen erfolgt.

Ein Gerät insbesondere für Personen mit einer Erkrankung oder Verletzung des Gleichgewichtsorganes mit der Merkmalen des Oberbegriffs des Anspruchs 1 geht aus CA-A1-2525 548 hervor. Mittels Sensoren wird stetig der Schwerpunkt der betreffenden Person ermittelt. Droht diese Person das Gleichgewicht zu verlieren und zu stürzen, wird ein Signal im Verhältnis zur Fallrichtung ausgesandt, um die Person anzuregen, seine Bewegung entsprechend zu korrigieren. Als Ersatz oder Ergänzung der Funktion des Gleichgewichtsorganes soll eine daran erkrankte Person vor Stürzen bewahrt werden. Es kann auch von Sportlern genutzt werden und diese vor Stürzen warnen. Mit dem Gerät können zwar Gleichgewichtsveränderungen erkannt und korrigiert werden, das Gerät ist jedoch nicht in der Lage eine nicht gewollte, falsche oder mangelhaft ausgeführte Bewegung der Beine, der Arme oder des Rumpfes zu erfassen und zu korrigieren, solange sich die betreffende Person im Gleichgewicht befindet. Zudem sieht das Gerät nicht vor, den Ausgabereiz von lähmungsbedingten Sensibilitätsunterschieden zu generieren, geschweige denn nach lähmungsbedingten Sensibilitätsunterschieden einzelner Körperregionen zu differenzieren.

Schließlich beschreibt DE 101 24 242 A1 eine Vorrichtung zur Haltungskontrolle einer Person bei der die mittels einer Sensoren aufweisenden Aufnahmeeinheit erfassten haltungsrelevanten Informationen durch eine Datenverarbeitungseinheit mit den Daten einer Expertendatenbank verglichen und so Haltungsabweichungen ermittelt werden, wobei die erforderliche Informationseinheit als Sender für optische und/oder akustische und/oder taktile Signale ausgebildet ist. Ein elektrostimulierendes Reizsignal kann nicht einmal für gefühlsarme Körperteile mit diesem Gerät nicht erzeugt werden.

Keines der genannten den Stand der Technik repräsentierenden Geräte ist für den Einsatz bei Personen mit gefühlsarmen Körperteilen und bei partiell gelähmten, insbesondere halbseitig gelähmten Personen zu deren Haltungs- und/oder Bewegungskorrektur geeignet. Die genannten Geräte können erfolgreich nur von Personen mit einer symmetrisch ausgebildeten Sensibilität ihrer Nerven benutzt werden und sind nicht dafür vorgesehen, die bei partiell gelähmten Personen gegebene lähmungsbedingte asymmetrisch ausgebildete Sensibilität ihrer Nerven auszugleichen und zu korrigieren. Die Asymmetrie der Sensibilität der Nerven unverzerrt wahrzunehmen und auszugleichen ist jedoch eine zwingende und unverzichtbare Vorbedingung für jede Art der Haltungs- und Bewegungskorrektur bei Personen mit gefühlsarmen Körperteilen und bei partiell gelähmten, insbesondere halbseitig gelähmten Personen. Diese Vorbedingung kann keines der Geräte nach dem Stand der Technik auf Grund ihres Aufbaues erfüllen.

Aufgabe der Erfindung ist es daher, Personen mit gefühlsarmen Körperteilen sowie partiell gelähmten, insbesondere halbseitig gelähmten Personen ein tragbares Gerät zur Verfügung zu stellen, das aufgrund von Wahmehmungsstörungen vorhandene Gangbild eines (geh-)behinderten Menschen an das eines "normalen" Gangbildes eines gesunden Menschen anzunähern.

Erfindungsgemäß wird die Aufgabe durch ein tragbares Gerät für eine Bewegungs- und/oder Haltungskorrektur von Personen mit gefühlsarmen Körperteilen sowie partiell gelähmten, insbesondere halbseitig Personen mit mindestens einem mit dem zu kontrollierenden Körperteil verbundenen Sensor gelöst, dessen zeitkontinuierliches Signal über eine erste Signalübertragungsstrecke einer Signalaufbereitungs- und - auswerteeinheit aufgeschaltet ist, die Mittel zur Umwandlung des Signals in ein Reizsignal aufweist, das über eine zweite Signalübertragungsstrecke mit mindestens einem taktilen und/oder elektrostimulierenden Reizgeber verbunden ist und der einen Reiz an dem zu kontrollierenden Körperteil erzeugt und so die Haltung und/oder Bewegung des Körperteiles oder der partiell gelähmten Person korrigiert, wobei das Gerät mindestens einen Sensor für einen gefühlsaktiven Köperteil besitzt, wobei fehlerhafte, durch Wahmehmungsstörungen der Person vorhandene Bewegungsabläufe erkannt werden und eine Unsymmetrie dadurch ausgeglichen wird, indem die Umwandlung des Signals des Sensors durch die Signalaufbereitungsund Auswerteeinheit in ein Reizsignal für den Reizgeber kontinuierlich erfolgt, der Reizgeber den Reiz am zu kontrollierenden Körperteil zeitnah zu dem vom Sensor abgegebenen Signal erzeugt, der den Reiz interaktiv bis zum Erreichen eines für das Körperteil veränderbar vorgegebenen Zielwertes erzeugt, bei Anordnung der Sensoren an einem paarweise vorhandenen Körperteil, die Sensoren an jeweils gleicher Stelle der paarigen Körperteile angeordnet ist, der Ausgleich der Unsymmetrie durch voneinander unabhängige Reize an jedem Körperteil durch Vorgabe einer Sollbewegung des gefühlsaktiven Körperteils erfolgt und der Reiz in seiner Intensität in Abhängigkeit vom Grad der Wahmehmungsstörung des Körperteiles einstellbar ist.

Vorteilhafte Ausgestaltungen des erfindungsgemäßen Gerätes ergeben sich aus den Merkmalen des Ansprüche 2 bis 11.

Mit dem erfindungsgemäßen Gerät wird die betroffene Person in die Lage versetzt, die bei gefühlsarmen Körperteilen oder bei partieller Lähmung vorhandene Wahmehmungsstörung auszugleichen. Es ist ein besonderer Vorzug der Erfindung, den Bewegungs- und/oder Haltungsdefekt des betreffenden Körperteiles in einer für die betreffende Person unbewusst ablaufenden Reaktion zu korrigieren.

Durch die Reize werden die jeweils zu bewegenden Muskeln zur Kontraktion angeregt und zwar konkret nach Ort und Zeit des gewünschten Bewegungsablaufes bzw. der Haltungskorrektur.

Es wird davon ausgegangen, dass sich die überaus positive Wirkung der Erfindung vor allem wie folgt erklärt werden könnte:
1. Personen mit gefühlsarmen Körperteilen und partiell gelähmte Personen nehmen Haltungen und Bewegungen je nach Körperhälfte und Lage des Körperteiles zum Gehirn im Unterbewusstsein asymmetrisch wahr, jedoch mit der Folge, dass sie subjektiv bewusst ihre schiefe Haltung oder ihren krummen oder humpelnden Gang als symmetrisch ausgeglichenen Gang beziehungsweise als eine symmetrisch aufrechte Haltung ansehen.
2. Impulse, die das Gehirn aussendet, werden bei diesen Personen abhängig von der Körperhälfte und der Lage des Körperteils zum Gehirn unterschiedlich oder auch gar nicht weitergeleitet. Bei halbseitig gelähmten Personen wird auch die Weitergabe von körpereigenen Informationen als unsymmetrisch angenommen.

Das erfindungsgemäße Gerät erlaubt es, diese Defizite zu überwinden.

Die genannten Überlegungen sind auch eine mögliche Erklärung für die Beobachtung, dass derart behinderte Personen, deren Gangbild sich im Verlauf einer Therapie mit dem erfindungsgemäßen Verfahren und Gerät deutlich verbessert hat, diese Verbesserung nicht selber wahrnehmen. Für diese Personen fühlt sich das neue und bessere Gangbild genauso ausgeglichen an, wie das vorherige schlechtere.

Mit der Erfindung wird der Zustand überwunden, dass es Personen mit gefühlsarmen Körperteilen und partiell gelähmten Personen, obwohl sie über Muskeln und Knochen bei den betreffenden Körperteilen verfügen, nicht möglich ist, normal zu gehen und zu stehen. Mit der Erfindung wird das hier bestehende Problem der Bewegungs- und Haltungssteuerung gelöst.

Ohne die Erfindung ist jede Steuerung von außen, die das Gehirn filtern muss (z.B. eine 2. Person, Videokamera, Spiegel, Ton, Licht) immer zeitverzögert und kommt damit zu spät oder gar nicht an dem zu korrigierenden Körperteil an. Zudem würde eine solche Steuerung. die durch das Gehirn gefiltert werden muss, unsymmetrisch an beide Körperhälften weitergegeben werden.

Bisher konnte physiotherapeutisch das Gangbild der betroffenen Personen zum Teil deutlich verbessert werden. Therapieerfolge sind aber, wie in der Fachliteratur immer wieder betont wird, nicht von Dauer. Der Grund hierfür wird darin gesehen, dass das Problem der Wahrnehmungsunsymmetrie und seine Auswirkungen auf das Gangbild und die Körperhaltung gelähmter Personen bisher nicht behandelt werden konnte. Mit der Erfindung wird ein wesentlicher Beitrag geleistet, diesen Zustand zu überwinden.

Damit Therapieerfolge vom Körper erinnert und dauerhaft gemacht werden können, dient die Erfindung darüber hinaus auch zur Konditionierung des vegetativen Nervensystems in Hinblick auf unbewusste Muskelaktivitäten des Bewegungs- und Haltungsapparates. Dabei geht es um die Aktivierung körpereigener Systeme, die von der Lähmung oder Gefühlsarmut nicht betroffen sind und die Steuerung des Bewegungs- und Haltungsapparates zumindest teilweise übernehmen können.

Dieser Lernvorgang ist identisch mit dem, bei dem der Körper auf einen Schmerz automatisch mit einer Änderung der Körperhaltung reagiert, um diesen Schmerz zu umgehen. Bei länger anhaltendem Schmerz verändert sich die Körperhaltung dauerhaft - auch wenn der Schmerz aufhört. Auf Dauer erlernt also der Körper mit einem solchen Gerät die eigenen Bewegungen auf objektive Symmetrie zu beurteilen.

Um eine zeitnahe und interaktive Korrektur der Haltung und/oder Bewegung des betreffenden Körperteiles leisten zu können, wird durch die Erfindung eine Beteiligung des Gehirns umgangen. Das bedeutet, dass Informationen über die Symmetrie zum Beispiel von Bewegungen synchron mit diesen Bewegungen gemeldet werden und zwar unmittelbar von dem Ort, an dem die jeweilige Bewegung erfolgt (z. B. Knie, Wirbelsäule, Schulter) und ein taktiler Reiz (vorzugsweise Schmerz), nahezu in Echtzeit und unabhängig vom Wollen der Person an der Stelle ausgeübt wird, die maßgeblich die Korrektur der Bewegung beeinflusst und zwar solange, bis die Korrektur das gewünschte Ergebnis zeigt. Das beinhaltet auch, dass die Information, wann eine mögliche Bewegungs- oder Haltungskorrektur erfolgreich ist, auch synchron mit der erfolgreichen Korrektur erfolgt.

Die Reizstärke ist gemäß einer vorteilhaften Ausgestaltung der Erfindung einstellbar, um die Wahrnehmungsunsymmetrie in einem Lernprozess ausgleichen zu können.

Wenn zwei Sensoren vorgesehen sind, können nicht nur die Signale der einzelnen Sensoren ausgewertet werden, sondern auch beispielsweise das Ergebnis einer vektoriellen Addition dieser Sensoren, was beispielsweise die Analyse von relativ zueinander erfolgenden Bewegungen ermöglicht.

Diesem Zweck dient unter anderem auch die Maßnahme, dass mindestens ein Sensor ein Beschleunigungssensor ist.

Der andere Sensor kann dabei ein Schalter sein. Ein solcher Schalter kann mit Vorteil auch dazu benutzt werden, dass sein Schaltsignal als Triggersignal zur Aufzeichnung und Darstellung der Bewegungsabläufe dient. Wenn mindestens ein Paar rechtwinklig zueinander messende Beschleunigungssensoren vorgesehen sind, können an dem Messort alle Bewegungsparameter gleichzeitig erfasst werden.

Bei zwei Paaren rechtwinklig zueinander messender Beschleunigungssensoren können auch durch geeignete mathematische Methoden Relativbewegungen analysiert werden.

Als Erkennungssignal wird eine Beschleunigung gemessen. Die Messung der Beschleunigung hat den Vorteil, dass aus ihr durch entsprechende mathematische Verarbeitung nicht nur die Beschleunigung selbst, sondern auch Aussagen über Geschwindigkeit und Ort getroffen werden können.

Wenn eine Beschleunigung in zwei zueinander senkrechten Richtungen gemessen wird, ist daraus sowohl ein Signal für die Neigung als auch der Vektor einer Bewegung erkennbar. Es können dadurch gleichgeartete Sensoren für unterschiedliche Aufgaben eingesetzt werden, was das Verfahren und dessen Verwirklichung erleichtert.

Dadurch, dass die Beschleunigung an unterschiedlichen Stellen, vorzugsweise gleichzeitig, gemessen wird, können mit geeigneten mathematischen Methoden auch relativ zueinander erfolgende Bewegung gemessen werden, beispielsweise eine Körperkrümmung oder die Winkelstellung des Beins.

Vorteilhafterweise ist vorgesehen, dass eine Integration der Beschleunigung zur Erzeugung eines Geschwindigkeitssignals erfolgt und/oder eine Integration des Geschwindigkeitssignals zur Erzeugung eines Wegsignals erfolgt.

Mit Vorteil kann man durch vektorielle Addition beispielsweise von Wegsignalen, ein Vergleichssignal erzeugen, das beispielsweise den Abstand des Fußes vom Hüftgelenk angibt.

Durch Vergleichsmessungen, bei denen die Signale an gesunden und kranken Gliedmaßen gemessen werden und dabei die zeitlich individuelle Verlaufsänderungen zur Erzeugung von Hüllkurven genützt werden, können Toleranzbänder individuell für das Training von kranken Gliedmaßen bestimmt werden. Wenn eine Festlegung eines Grenzkurvenverlaufs, vorzugsweise automatisch erfolgt, können durch Vergleich der momentanen Bewegungsparameter mit den Grenzkurven Ereignisse erzeugt werden, die dann die durch Reize die entsprechenden Körperteile stimulieren.

Dazu ist mit Vorteil vorgesehen, dass der an einem kranken Glied gemessene Signalverlauf mit dem Grenzkurvenlauf verglichen wird und in Abhängigkeit vom Ergebnis dieses Vergleichs ein Reizsignal erzeugt wird, das vorzugsweise in Abhängigkeit der Größe des Abweichens moduliert wird. Somit kann in der Therapie auch eine zusätzliche Information durch die unterschiedliche Stärke des Reizes gegeben werden, die anzeigt, wie groß die Abweichung von dem gewünschten idealen Bewegungsablauf ist.

Wenn unterschiedliche Ausgangskanäle zur Erzeugung von Reizsignalen in Abhängigkeit von Ort und/oder Stärke der Abweichung angesprochen werden, können die Reize nach Art und Stärke und Ort individuell an die Schädigungen des Patienten angepasst werden.

Dem dient auch die Maßnahme, dass mehrere Eingangskanäle von unterschiedlichen Sensoren gespeist werden, wobei jedem Eingangssignalkanal unterschiedliche Grenzkurven zugeordnet sind.

Wenn mindestens ein Neigungssensor vorgesehen ist, kann über die Schulterneigung im Vergleich zur Hüftneigung auch eine Krümmung der Wirbelsäule auf einfache Weise detektiert werden.

Der geeigneten Analyse bei unterschiedlichen Krankheitsbildern dient auch die Maßnahme, dass mindestens ein Sensor am Körper und mindestens ein Sensor an einem gefühlsarmen Gliedmaß befestigt sind.

Besonders einfach gestaltet sich die Applikation der Sensoren, wenn mindestens ein Sensor am Kleidungsstück eines Patienten befestigt ist. Der Patient muss dann nur das Kleidungsstück anziehen. In diesem Kleidungsstück können sich auch die Auswerteelektronik und zusätzliche Energiequellen zur Versorgung der Sensoren und der Auswerteelektronik sowie geeigneter Transponder für die Messdatenübermittlung enthalten sein.

Die Messsignalverarbeitung und -auswertung ist besonders sicher und universell leicht anpassbar, wenn die Auswerteelektronik als PDA mit geeigneter Auswertesoftware ausgebildet ist.

Die Betriebsdauer des Gerätes lässt sich fast beliebig einstellen, wenn dem Gerät eine getrennte Energiequelle, z. B. Batterie oder Brennstoffzelle, zugeordnet ist.

Der Tragekomfort erhöht sich vorteilhaft, wenn mindestens eine der Wirkverbindungen als drahtlose Verbindung ausgebildet ist, z. B. Blue-Tooth - oder WLAN ― oder IR-Verbindung.

Dadurch, dass die Auswerteelektronik eine Verbindung zu einer bildgebenden Ausgabeeinheit aufweist, kann der behandelnde Arzt visuell direkt Fehler im Bewegungsablauf erkennen und die dafür notwendigen Tolerenzbänder für die Bewegung zur Erzeugung von Reizsignalen individuell anpassen.

Besonders universell wird das Gerät, wenn die Auswerte- und Visualisierungsaufgaben gerätetechnisch getrennt werden, in dem Auswerteelektronik eine dritte Wirkverbindung zu einem Kontrollgerät z. B. eines Trainers, und/oder einer Speichereinheit aufweist. Dies Kontrollgerät kann beispielsweise dazu dienen, die individuellen Toleranzbänder zunächst festzulegen und in einem PDA abzuspeichern, der dann unabhängig vom Kontrollgerät die Auswertung und Erzeugung von Reizen übernimmt.

Die individuelle Anpassung an den Patienten wird dadurch erleichtert, dass die Auswerteelektronik Vergleichsparameter Grenzkurven aufweist, die vorzugsweise während des Trainings insbesondere über die dritte Wirkverbindung vom Kontrollgerät veränderbar ausgebildet sind. Dadurch lassen sich noch während des Anpassungsvorgangs auch die Grenzkurven, die zur Erzeugung der Reizsignale herangezogen werden, anzupassen.

Sollten vom gefühlsarmen Körperteil Reize nur unzureichend bemerkt werden, so kann es in bestimmten Fällen vorteilhaft sein, wenn mindestens ein Aktuator mit dem gefühlsarmen Körperteil verbunden ist, um die notwendige Bewegung zwangsweise zu erzeugen und dadurch einen Lerneffekt zu erzielen.

Erfindungsgemäß lassen sich die Toleranzbilder bestimmen, wenn mindestens ein zusätzlicher Sensor an einem gefühlsaktiven Körperteil vorgesehen ist. Dieser Sensor, der dann an gleicher Stelle des anderen paarigen Körperteils angeordnet ist, kann bei der Bewegung die gewünschte Sollbewegung vorgeben, die dann der Erzeugung von Reizen für den kranken Körperteil dient. Beispielsweise können am Unterschenkel an gleicher Stelle der beiden Beine solche Sensoren angeordnet werden. Bei halbseitiger Lähmung kann dann der Sensor am gesunden Bein die Bewegungskurve vorgeben, die dann das kranke Bein innerhalb eines Tolerenzbandes nachahmen soll.

Eine störende Verdrahtung am Körper des Patienten lässt sich vermeiden, wenn der mindestens eine Sensor und/oder das mindestens eine Mittel zur Erzeugung eines Reizes mittels eines Transponders mit Energie versorgt wird.

Die Erfindung wird anhand einer bevorzugten Ausführungsform beispielhaft erläutert. Es zeigen
**Fig. 1** schematisch das Messsystem in Blöcken dargestellt einschließlich der Applikationen am Patienten,
**Fig. 2** eine vergrößerte Darstellung des Patienten mit möglichen Applikationspunkten von Sensoren und Reizgebern,
**Fig. 3** ein schematisches Wegdiagramm mehrerer Schritte und
**Fig. 4** ein Weg - Zeit - Diagramm mehrerer Schritte.

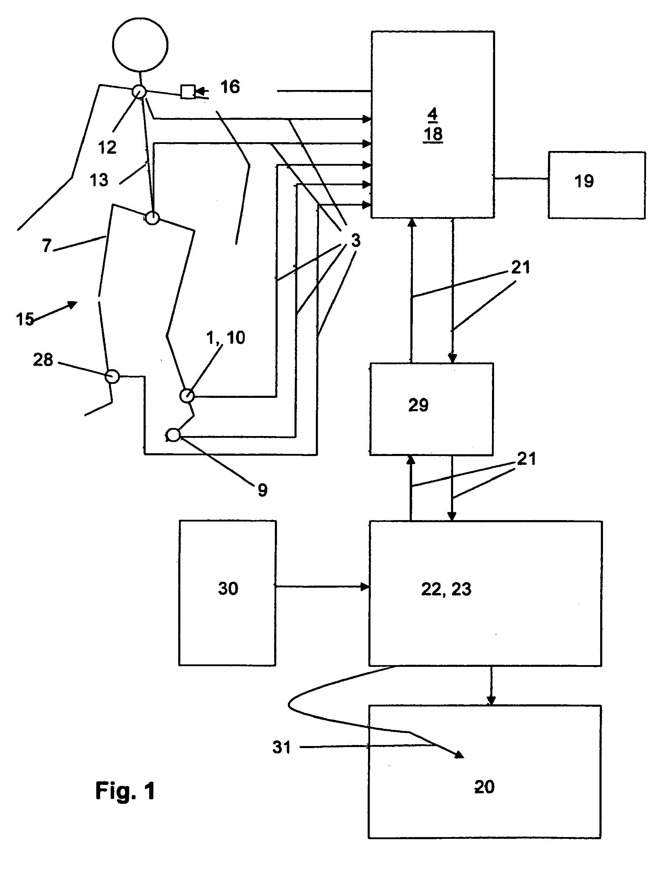

In **Fig. 1** ist in Strichdarstellung der Patient 15 gezeigt, der an verschiedenen Stellen als Kreis dargestellte Sensoren 1, 10, 9, 12, 11, 30 trägt. Die Reizquellen sind als Rechtecke 6, 17, 16, 18, 19 dargestellt. Von den Sensoren gehen Wirkverbindungen 3 zur Auswerteelektronik 4, die vorzugsweise ein PDA ist, mit einer zusätzlichen Energiequelle 21. Auf diesem PDA wird ein geeignetes Auswerteprogramm betrieben, das in Abhängigkeit der Sensorsignale dieser Wirkverbindungen 5 angesteuert werden. Beispielsweise kann das Mittel 6 zur Erzeugung eines Reizes ein taktiler oder elektrostimulierender Reizgeber 16, 17 sein.

Die vorstehenden Reizgeber umgehen mit der von ihnen erzeugten Wirkung gewissermaßen das Gehirn. Unter bestimmten Umständen, können auch Aktuatoren zur Anwendung kommen, so dass bei Bedarf ein Aktuator 8 angesteuert wird, der zwischen dem Fuß und Unterschenkel des Patienten wirkt. Zwar sind die Sensoren vorzugsweise als Beschleunigungssensoren ausgebildet, es können aber auch Schalter 9 oder Winkel-, Abstands- oder Drehbewegungsänderungen erfassende Sensoren bekannter Art zum Einsatz kommen. In **Fig. 1** ist beispielsweise ein Schalter 9 am Fuße des Patienten dargestellt. Über diesen Schalter lässt sich auch die Messwertauswertung oder die Darstellung graphischer Verläufe leicht triggern.

Über eine drahtlose WLAN-Verbindung 21 sendet der PDA 18 die Messsignale über den Router 29 an ein Kontrollgerät 22, beispielsweise einem Laptop, der auch den nötigen Datenspeicher 23 beinhaltet und auf dem Bildschirm 20 die Messdaten in geeigneter Weise darstellt. Die notwendigen Patientenangaben und die Parameterangaben können über eine Tastatur 30 dem Kontrollgerät 22 eingegeben werden. Mittels des graphischen Eingabegerätes 31 lassen sich die Toleranzbereiche auch graphisch leicht an dem Bildschirm 20 eingeben.

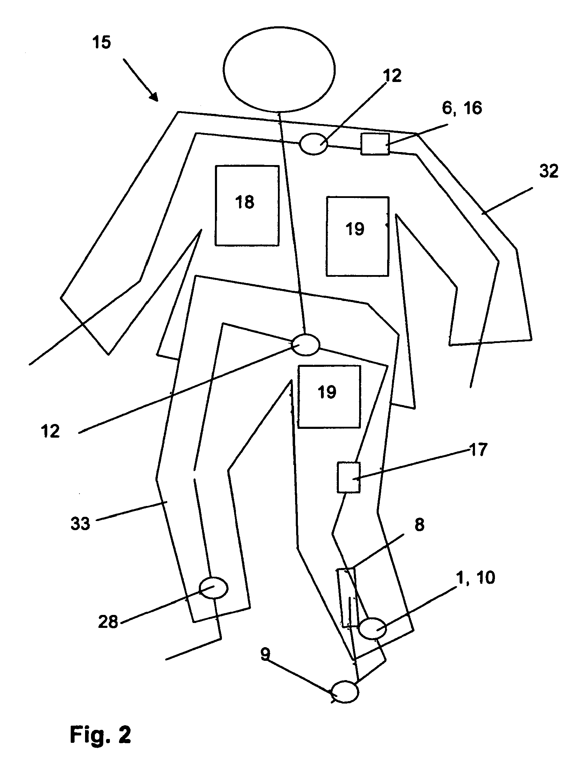

**Fig. 2** zeigt bevorzugte Orte zur Anbringung von Sensoren bzw. Reizgebern. Beispielsweise ist ein Neigungssensor 12 im Kragen einer Jacke 32 eingenäht, so dass dieser Sensor die Neigung der Schulter des Patienten erfassen kann. Ein weiterer Neigungssensor 12 ist beispielsweise im Gürtel einer Hose des Patienten eingearbeitet, so dass die Beckenneigung des Patienten von diesem Neigungssensor erfasst werden kann. Aus den Neigungswinkeln und der Beckenbreite bzw. Schulterbreite, können die Verlagerungen der Hüftgelenke und des Armgelenks errechnet werden. Auf diese Weise kann auch die Krümmung der Wirbelsäule gemessen werden. Mit Hilfe des im Hosenbein 33 eingenähten Beschleunigungssensorpaares 28 kann die Bewegung beispielsweise des gesunden Beins erfasst werden. Das Sensorpaar 1 misst die Bewegung des kranken Beines bei einem halbseitig gelähmten Patienten. Der taktile Reizgeber, beispielsweise ein Vibrator 16, signalisiert der Signalverarbeitungs- und -auswerteeinheit 4, dass die Schulter einer Korrektur bedarf. Der Elektrostimulationsgeber 17 signalisiert, dass die Beinstellung zu ändern ist. Die Sensoren und Reizgeber können innerhalb der Kleidung verdrahtet werden. Es ist aber auch eine gruppenweise oder einzelne Verschaltung der Sensoren und Signalgeber mittels Funkverbindung, beispielsweise durch Blue-Tooth, denkbar. Zu diesem Zweck sind zusätzliche Energiequellen 19 in der Jacke bzw. Hose des Patienten eingearbeitet.

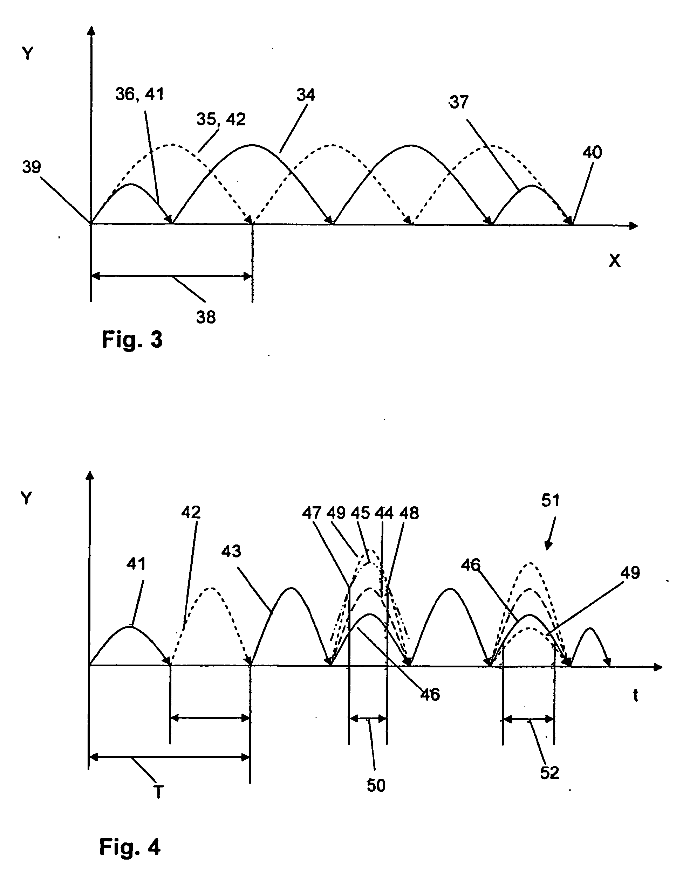

**Fig. 3** stellt schematisch den Bewegungsablauf der Fußgelenke eines Patienten dar, wobei derer Weg des gesunden Beines mit Ziffer 6 und der des gelähmten Beines mit 35 bezeichnet ist. Dabei ist die Y-Koordinate die Höhe des Fußes über dem Boden und X-Koordinate der Abstand von der Anfangsstellung. Der Anfangsschritt 38 und der Endschritt 37 haben die halbe Schrittweite 38. Zum Anfangspunkt 39 und Endpunkt 40 sind die Füße parallel und der Patient steht.

**Fig. 4** zeigt den Bewegungsablauf gemäß **Fig. 3** in einem zeitabhängigen Diagramm. Die Abszisse ist mit "t" bezeichnet, die Ordinate stellt wiederum die Höhe über dem Fußbodenniveau dar. Das gesunde Bein 41 und das gelähmte Bein 42 bewegen sich abwechselnd im Rhythmus der halben Periode t. Aus einer Halbperiode 43, die die Bewegung des gesunden Beines darstellt, wird die Sollbewegung der folgenden Halbperiode des gelähmten Beines errechnet, die durch die unterbrochen gezeichnete Linie 44 dargestellt ist. Um diese Linie wird ein Toleranzband gelegt, dessen obere Grenze 45 und untere Grenze 46 den Bewegungsablauf des gelähmten Beines darstellen. In den Punkten 47 und 48, die Ereignisse des Überschreitens signalisieren, bzw. des Unterschreitens signalisieren, schneidet der tatsächliche Bewegungsablauf 49 die Toleranzkurve 45 und die Auswerteelektronik gibt einen Reiz an den Elektrostimulationsgeber 17. Im Punkt 48 unterschreitet wiederum der tatsächliche Bewegungsablauf 49 die obere Toleranzkurve 45, so dass der Reizgeber abschaltet. Für die durch den Fall 50 darstellte Dauer erfährt somit der Körperteil einen Reiz, der ihn sozusagen zu einer Verbesserung seine Bewegung veranlasst, obwohl er subjektiv seine defekte Bewegung als normal angesehen hat. In der Halbperiode 51 unterschreitet der Bewegungsablauf 49 die untere Toleranzgrenze 46, so dass das Signal entweder für die gesamte Dauer dieser Halbperiode ein Reizsignal generiert wird, oder aber in Abhängigkeit eines Schwellwertes nur für die Dauer der durch den Fall 52 dargestellten Zeit.

Das Gerät kann nicht nur bei Patienten eingesetzt werden. Auch Sportler können ihre Bewegungen mit Hilfe des Gerätes kontrollieren und selbst geringe Unsymmetrien in ihren Haltungen und/oder Bewegungen korrigieren. Auch pädagogisch lässt sich das Gerät zur Darstellung und Analyse von Bewegungsabläufen einsetzen. Statt der Beschleunigungssensoren können auch direkt den Ort erfassende Sensoren zur Anwendung gelangen, beispielsweise optische Marken, die von Bildverarbeitungssoftware erfasst werden, oder Sensoren, die durch Laufzeitmessungen den Ort errechnen. Sogar Differenzial-GPS-Systeme sind im Sinne der Erfindung zur Bestimmung des Bewegungsverlaufs einsetzbar.

Der Programmablauf kann sich beispielsweise im Wesentlichen wie folgt gestalten:

### Start

### 1.Schritt

Parametereingabe:
Toleranzbereiche Tx, Ty, BV, KK
Hüftbreite BH
Schulterbreite BS
Empfindlichkeit bzw. Schwellwert kx, ky
Vorgesehene Sensoreingänge für
Ort gesunder Fuß
Ort kranker Fuß
Hüftneigung
Schulterneigung

### 2. Schritt

Ermittlung der Werte für
Schrittfrequenz f
Schrittweite S

Messungen am gesunden Bein
Abfrage ax > kx oder ay > ky
Wenn nein, dann warten
wenn ja, dann weiter

Einlesen der Sensorwerte Beschleunigung ax und ay
Integratinon von ax, ay zur Geschwindigkeit vx, vy
Intergartion von vx, vy zum Ort x,y
Einlesen und Speichern der Zeit t0

wenn ja, dann zurück

Wenn nein, dann Speichern der Zeit t1
Berechnen der Werte
Schrittfrequenz f
Schrittweite S

### 3. Schritt

Messungen am kranken Bein
Berechnen der Sollkoordinaten des kranken Beines zum Zeitpunkt t+1/f
Speichern der Sollkoordinaten als Vektor (xs,vs,ts)

Messung am Körper
Einlesen des Sensorwertes phiH
Einlesen des Sensorwertes phiS
Berechnen der Körperkrümmung
Berechnen der Beinverkürzung

Einlesen der Sensorwerte Beschleunigung ax und ay
Integratinon von ax, ay zur Geschwindigkeit vx, vy
Intergartion von vx, vy zum Ort x,y

### 4. Schritt:

Generierung von Reizsignalen

Abfrage Ist-Koordinaten - Sollkoordinaten > Toleranzbereich?
Wenn nein, dann weiter
Wenn ja, dann Reizsignal auf Reizkanal 1 ausgeben

Abfrage Ist-Körperkrümmung - SollKörperkrümmung > Toleranzbereich?
Wenn nein, dann weiter
Wenn ja, dann Reizsignal auf Reizkanal 2 ausgeben

Abfrage Ist-Beinverkürzung - Sollbeinverkürzung > Toleranzbereich?
Wenn nein, dann weiter
Wenn ja, dann Reizsignal auf Reizkanal 3 ausgeben

### 5. Schritt

### Visualisierung

Anzeige der Messwerte im getriggerten Ortsfenster "Fußkoordinaten" in Abhängigkeit vom Ort
Anzeige der Sollwerte und der Messwerte des kranken Beines im laufenden Zeitfenster "Fußkoordinaten" in Abhängigkeit von der Zeit
Anzeige des Reizsignalverlaufs im laufenden Zeitfenster
Anzeige der Sollwerte und der Messwerte des kranken Beines im getriggerten Zeitfenster "Fußkoordinaten" in Abhängigkeit von der Zeit
Anzeige des Reizsignalverlaufs im laufenden Zeitfenster
Anzeigefelder für Patentendaten, wie Alter, Datum, Adresse, Arzt, Trainingszyklus Anzeigefelder für Parameter momentane Schrittweite, Schrittfrequenz
Anzeigefelder für Toleranzbereiche
Stellfenster für Toleranzbereiche

### 6.Schritt

### Protokollierung

Geeignete Datenaggregation und -Kompression, Speicherung eines Trainings auf CD als Variable der Zeit Speicherung der angezeigten Daten und Druck auf Befehl
Ende

### BEZUGSZEICHENAUFSTELLUNG

1. Sensor
2. gefühlsarmes Körperteil
3. Wirkverbindung
4. Auswerteelektronik
5. Wirkverbindung
6. Mittel zur Erzeugung eines Reizes
7. gefühlsaktiver Körperteil
8. Aktuator
9. Sensor, Schalter
10. Beschleunigunssensorpaar
11. Beschleunigunssensorpaar
12. Neigungssensor
13. Körper
14. Keidungsstück
15. Patient
16. taktiler Geber
17. Elektrostimulationsgeber
18. Personal Digital Assistent (PDA)
19. Energiequelle
20. Bildschirm
21. Wirkverbindung
22. Kontrollgerät
23. Speichereinheit
24. Vergleichsparameter
25. Grenzkurve
26. dritte Wirkverbindung
27. zusätzlicher Sensor
28. Sensor gesundes Bein
29. Router
30. Tastatur
31. graphisches Eingabegerät
32. Jacke
33. Hose
34. Weg gesundes Bein
35. Weg gelähmtes Bein
36. Anfangsschritt
37. Endschritt
38. Schrittweite
39. Anfangspunkt
40. Endpunkt
41. gesundes Bein
42. gelähmtes Bein
43. Halbperiode
44. Linie
45. obere Grenze
46. untere Grenze
47. Ereignis
48. Ereignis
49. Bewegungsablauf
50. Pfeil
51. Halbperiode
52. Pfeil

## Patentansprüche

1. Tragbares Gerät für eine Bewegungs- und/oder Haltungskorrektur von gefühlsarmen Körperteilen (2) und von partiell gelähmten Personen zur Verbesserung eines Haltungs- und/oder Gangbildes mit mindestens einem mit dem zu kontrollierenden Körperteil (2) verbundenen Sensor (1) dessen zeitkontinuierliches Signal über eine erste Signalübertragungsstrecke (3) einer Signalaufbereitungs- und -auswerteeinheit (4) ausgeschaltet ist und die Mittel zur Umwandlung eines Signals in ein Reizsignal aufweist, das über eine zweite Signalübertragungsstrecke (5) mit mindestens einem taktilen und/oder elektrostimulierenden Reizgeber (16; 17) verbunden ist, der einen Reiz an dem zu kontrollierenden Körperteil (2) erzeugt und so die Haltung und/oder Bewegung des Körperteiles (2) oder der partiell gelähmten Person korrigiert,
**dadurch gekennzeichnet, dass**
das Gerät mindestens einen Sensor (28) für einen gefühlsaktiven Köperteil besitzt,wobei fehlerhafte, durch Wahmehmungsstörungen der Person vorhandene Bewegungsabläufe erkannt werden und eine Unsymmetrie dadurch ausgeglichen wird, indem
die Umwandlung des Signals des Sensors (1) durch die Signalaufbereitungsund Auswerteeinheit (4) in ein Reizsignal für den Reizgeber (16; 17) kontinuierlich erfolgt;
der Reizgeber (16; 17) den Reiz am zu kontrollierenden Körperteil (2) zeitnah zu dem vom Sensor (1) abgegebenen Signal erzeugt,
der Reizgeber (16;17) den Reiz interaktiv bis zum Erreichen eines für das Körperteil (2) veränderbar vorgegebenen Zielwertes erzeugt,
bei Anordnung der Sensoren an einem paarweise vorhandenen Körperteil (2), die Sensoren an jeweils gleicher Stelle der paarigen Körperteile angeordnet sind, wobei mindestens ein Sensor an dem gefühlsaktiven und mindestens ein Sensor an dem gefühlsinaktiven der paarigen Körperteile angeordnet ist, der Ausgleich der Unsymmetrie durch voneinander unabhängige Reize an jedem Körperteil (2) durch Vorgabe einer Sollbewegung des gefühlsaktiven Körperteiles (2) erfolgt und
der Reiz in seiner Intensität in Abhängigkeit vom Grad der Wahmehmungsstörung des Körperteiles (2) einstellbar ist.

2. Gerät nach Anspruch 1, **dadurch gekennzeichnet, dass** das Reizsignal innerhalb vorgegebener Grenzen in Abhängigkeit von der Stärke des vom Sensor (1) erzeugten Signals modulierbar ist.

3. Gerät nach Anspruch 1 oder 2, **dadurch gekennzeichnet, dass** mindestens zwei, vorzugsweise identische Sensoren (1; 9) vorgesehen sind, die unabhängig voneinander Messsignale erzeugen und/oder der Sensor (1) ein Beschleunigungssensor und/oder ein Neigungssensor und/oder eine Winkel-, Abstands- oder Drehbewegungsänderung messender Sensor bekannter Art ist und/oder ein Sensor ein Schalter (9) ist.

4. Gerät nach einem der Ansprüche 1 bis 3, **dadurch gekennzeichnet, dass** Beginn und Ende einer Messung am Bein durch einen Schalter oder einen als Schalter wirkenden Beschleunigungs-, Kraft- oder Drucksensor bestimmt sind.

5. Gerät nach einem der Ansprüche 1 bis 4, **dadurch gekennzeichnet, dass** mindestens ein Paar (10) rechtwinklig zueinander messender Beschleunigungssensoren vorgesehen ist.

6. Gerät nach einem der Ansprüche 1 bis 5, **dadurch gekennzeichnet, dass** das Mittel zur Auswertung des Signals eine Auswerteelektronik (4), vorzugsweise ein PDA (Minicomputer) (18) mit einer geeigneten Auswertesoftware ist.

7. Gerät nach einem der Ansprüche 1 bis 6, **dadurch gekennzeichnet dass** mindestens eine Signalübertragungsstrecke (3; 5) als eine drahtlose Verbindung, vorzugsweise als Bluetooth-, WLAN- oder IR-Verbindung ausgebildet ist.

8. Gerät nach einem der Ansprüche 1 bis 7, **dadurch gekennzeichnet, dass** die Mittel (4) zur Auswertung des Signals mit einer bildgebenden Auswerteeinheit (20) verbunden sind.

9. Gerät nach einem der Ansprüche 1 bis 8, **dadurch gekennzeichnet, dass** die Signalaufbereitungs- und -auswerteeinheit (4) eine dritteSignalübertragungsstrecke (26) zu einem Kontrollgerät (22), zum Beispiel eines Trainers, und/oder einer Speichereinheit (23) aufweist.

10. Gerät nach einem der Ansprüche 1 bis 9, **dadurch gekennzeichnet, dass** die Signalaufbereitungs- und -auswerteeinheit (4) Vergleichsparameter (24) oder Grenzkurven bzw. Toleranzbereiche (25) und/oder Zielvorgaben aufweist, die vorzugsweise über die dritte Signalübertragungsstrecke (26) vom Kontrollgerät (22) veränderbar ausgebildet sind.

11. Gerät nach einem der Ansprüche 1 bis 10, **dadurch gekennzeichnet dass** dem Gerät eine getrennte Energiequelle, zum Beispiel eine Batterie oder Brennstoffzelle, zugeordnet ist und/oder ein Transponder den Sensor und/oder das Mittel zur Erzeugung des Reizes mit Energie versorgt.

## Claims

1. Portable device for correcting a movement and/or a posture of insensitive body parts (2) and of partially paralyzed persons to improve posture and/or gait, with at least one sensor (1) being connected to the body part (2) to be controlled and providing a continuous time signal which is entered into a signal processing and evaluation unit (4) via a first signal transmission path (3) having means for converting a signal into a stimulus signal, which is connected via a second signal transmission path (5) to at least one tactile and/or electrically stimulating stimulator (16; 17) creating a stimulus at the body part (2) to be controlled in order to correct a posture and/or movement of said partially paralyzed person,
**characterized in that**
said device has at least one sensor (28) for a sensitive body part, wherein abnormal sequences of motions caused by a person's sensory disorder are detected and an unbalance is eliminated by
converting the signal from said sensor (1) into a stimulating signal to said stimulator (16; 17) by said signal processing and evaluation unit (4);
having said stimulator (16; 17) generate said stimulus at the body part (2) to be controlled immediately after said signal was provided by said sensor (1),
having said stimulator (16; 17) generate said stimulus interactively until a target value is reached which may be variably set for the respective body part (2),
if sensors are positioned at a pair of body parts (2), placing said sensors at both of said pair of body parts in the same position, respectively, wherein at least one sensor is positioned at said sensitive one and at least one sensor is positioned at said insensitive one of said pair of body parts, wherein said imbalance is eliminated by independent stimuli at each of said body parts (2) using a preset target movement of said sensitive body part (2), and
the intensity of said stimulus being adjustable on the basis of the degree of sensory disorder of said body part (2).

2. Device according to claim 1, **characterized in that** said stimulating signal can be modulated within predefined limits based on the strength of the signal generated by said sensor (1).

3. Device according to claim 1 or claim 2, **characterized in that** at least two preferably identical sensors (1; 9) are provided for independently generating measuring signals, and/or said sensor (1) is at least one of a well-known accelerating sensor, inclination sensor, and sensor for measuring at least one of an angular change, a change of distance and a change of rotational movement, and/or one of said sensors is a switch (9).

4. Device according to one of claims 1 to 3, **characterized in that** the start and the end of a leg measurement is determined by a switch or by an acceleration, force or pressure sensor acting as a switch.

5. Device according to one of claims 1 to 4, **characterized in that** at least one pair (10) of acceleration sensors is provided which perform orthogonal measurements with respect to one another.

6. Device according to one of claims 1 to 5, **characterized by** said signal evaluation means comprising evaluation electronics (4), preferably a PDA (minicomputer) (18) provided with a suitable evaluation software.

7. Device according to one of claims 1 to 6, **characterized by** said at least one signal transmission path (3; 5) being configured as a wireless connection, preferably a Bluetooth, WLAN or IR connection.

8. Device according to one of claims 1 to 7, **characterized by** said signal evaluation means (4) being connected to an imaging evaluation unit (20).

9. Device according to one of claims 1 to 8, **characterized by** said signal processing and evaluation unit (4) having a third signal transmission path (26) to a control device (22), for example of a trainer, and/or a storage unit (23).

10. Device according to one of claims 1 to 9, **characterized by** said signal processing and evaluation unit (4) having comparison parameters (24) or limit curves or tolerance ranges (25), respectively, and/or target values which are preferably variably provided by said control device (22) via said third signal transmission path (26).

11. Device according to one of claims 1 to 10, **characterized by** said device having its own separate energy source, for example a battery or a fuel cell, and/or by a transponder supplying energy to said sensor and/or said means for generating said stimulus.

## Revendications

1. Dispositif portable de contrôle du mouvement ou de la position de parties du corps (2) présentant une sensibilité réduite et de personnes partiellement paralysées destiné à améliorer la position et/ou la démarche, comportant au moins un capteur (1) relié à la partie du corps (2) à contrôler et dont le signal continu dans le temps est appliqué via un premier trajet de transmission du signal (3) à une unité de traitement et d'évaluation de signaux (4) et présente des moyens permettant la conversion du signal en un signal stimulant relié via un deuxième trajet de transmission du signal (5) à au moins un transmetteur de stimuli tactile et/ou électrostimulant (16 ; 17) qui engendre un stimulus sur la partie du corps (2) à contrôler et corrige ainsi la position et/ou le mouvement de la partie du corps (2) ou de la personne partiellement paralysée,
**caractérisé en ce que**
le dispositif est doté d'au moins un capteur (28) pour une partie sensible du corps détectant les mouvements incorrects dus à des troubles sensoriels de la personne et compensant l'asymétrie éventuelle par le fait que l'unité de traitement et d'évaluation du signal (4) convertit de façon continue le signal du capteur (1) en un signal stimulant pour le transmetteur de stimuli (16, 17) ;
le transmetteur de stimuli (16, 17) engendre le stimulus sur la partie du corps (2) à contrôler quasi simultanément au signal émanant du capteur (1),
le transmetteur de stimuli (16, 17) engrendre le stimulus de façon continue jusqu'à ce qu'une valeur cible prédéfinie modifiable pour la partie du corps soit atteinte,
que, lorsque les capteurs sont disposés sur des partie du corps symmétriques (2), les capteurs sont placés au même point des partie du corps à paires, au moins un capteur étant disposé sur la partie sensible et au moins un capteur étant placé sur la partie insensible des partie du corps à paires,
la compensation de l'asymétrie s'effectuant par des stimuli indépendants appliqués à chacune des parties du corps (2) en imposant le mouvement désiré de la partie du corps sensible (2), et
l'intensité du stimulus étant réglable en fonction du degré du trouble sensoriel de la partie du corps (2).

2. Dispositif selon la revendication 1, **caractérisé en ce que** le signal stimulant peut être modulé dans des limites prédéfinies en fonction de l'intensité du signal généré par le capteur (1).

3. Dispositif selon la revendication 1 ou 2, **caractérisé en ce qu'**il comporte au moins deux capteurs (1, 9) de préférence identiques qui génèrent des signaux de mesure indépendants l'un de l'autre et/ou que le capteur (1) est un capteur d'accélération et/ou un capteur d'inclinaison et/ou un capteur de type connu mesurant une modification de l'angle, de l'écart ou du mouvement rotatoire et/ou qu'un capteur est un commutateur (9).

4. Dispositif selon l'une des revendications 1 à 3, **caractérisé en ce que** le début et la fin d'une mesure au niveau de la jambe sont déterminés par un capteur d'accélération, d'effort ou de pression faisant office de commutateur.

5. Dispositif selon l'une des revendications 1 à 4, **caractérisé en ce qu'**il prévoit au moins deux capteurs d'accélération (10) effectuant des mesures perpendiculaires l'une par rapport à l'autre.

6. Dispositif selon l'une des revendications 1 à 5, **caractérisé en ce que** le moyen d'évaluation du signal est un appareil électronique d'évaluation (4), de préférence un PDA (ordinateur de poche) (18) doté d'un logiciel d'évaluation approprié.

7. Dispositif selon l'une des revendications 1 à 6, **caractérisé en ce qu'**au moins un trajet de transmission du signal (3, 5) est une connexion sans fil telle qu'une connexion Bluetooth, Wi-Fi ou infrarouge.

8. Dispositif selon l'une des revendications 1 à 7, **caractérisé en ce que** les moyens (4) d'évaluation du signal sont connectés à une unité d'évaluation à imagerie (20).

9. Dispositif selon l'une des revendications 1 à 8, **caractérisé en ce que** l'unité de traitement et d'évaluation des signaux (4) présente un troisième trajet de transmission des signaux (26) vers un appareil de contrôle (22), par exemple d'un entraîneur, et/ou vers une unité de mémoire (23).

10. Dispositif selon l'une des revendications 1 à 9, **caractérisé en ce que** l'unité de traitement et d'évaluation des signaux (4) présente des paramètres de comparaison (24) ou des courbes limites ou zones de tolérance (25) et/ou des objectifs indiqués de façon modifiable par l'appareil de contrôle (22) de préférence via le troisième trajet de transmission des signaux (26).

11. Dispositif selon l'une des revendications 1 à 10, **caractérisé en ce qu'**il dispose d'une source d'énergie séparée, telle qu'une pile ou cellule à combustible, et/ou qu'un transpondeur alimente en énergie le capteur et/ou le moyen de génération du stimulus.
